# EUROPEAN PATENT SPECIFICATION

(11) **EP 1 498 135 B1**
(45) Date of publication and mention of the grant of the patent: **26.12.2012**
(21) Application number: 03715700.5
(22) Date of filing: 01.04.2003
(51) Int. Cl.: A61K 38/22, A61K 47/32, A61K 47/36, A61K 47/38, A61P 1/02, A61K 9/00

(54) **DENTAL VISCOUS PHARMACEUTICAL CONTAINING BASIC FIBROBLAST GROWTH FACTOR**
DENTALE VISKOSE PHARMAZEUTIKA MIT BASISCHEM FIBROBLASTEN-WACHSTUMSFAKTOR
PREPARATION PHARMACEUTIQUE DENTAIRE VISQUEUSE CONTENANT UN FACTEUR DE CROISSANCE DES FIBROBLASTES

(30) Priority: 01.04.2002 JP 2002098977
(43) Date of publication of application: 19.01.2005
(73) Proprietor: KAKEN PHARMACEUTICAL CO., LTD., Tokyo 113-8650 (JP)
(72) Inventor: FUKUNAGA, Kazuhiro, Central Research Laboratories, Fujieda-shi, Shizuoka 426-8646 (JP); OGATA, Yuji, Kaken Pharmaceutical Co., Ltd., Tokyo 113-8650 (JP); FURUKAWA, Akihiko, Kaken Pharmaceutical Co., Ltd., Tokyo 113-8650 (JP); KONNO, Yoshihiro, Central Research Laboratories, Fujieda-shi, Shizuoka 426-8646 (JP)
(74) Representative: HOFFMANN EITLE
(86) International application number: PCT/JP2003/004166
(87) International publication number: WO 2003/082321

(56) References cited:
- EP-A1- 0 256 611
- EP-A1- 0 677 294
- EP-A1- 0 702 959
- WO-A1-94/06399
- JP-A- 8 295 637
- TABATA YASUHIKO ET AL: "Biodegradation of hydrogel carrier incorporating fibroblast growth factor" TISSUE ENGINEERING, LARCHMONT, NY, US, vol. 5, no. 2, 1 April 1999 (1999-04-01), pages 127-138, XP002568999 ISSN: 1076-3279
- DATABASE WPI Week 199513 Thomson Scientific, London, GB; AN 1995-093773 XP002577913 & JP 07 017876 A (KAKEN PHARM CO LTD) 20 January 1995 (1995-01-20)

## Description

### Technical field

The present invention relates to a viscous preparation for dental use containing basic fibroblast growth factor and can be used for treatment of various kinds of periodontal diseases such as periodontosis, etc.

### Background art

bFGF, i.e., basic fibroblast growth factor has been found as a protein which markedly promotes growth of fibroblast. After that, it has been clarified *in vitro* that it not only promotes growth of the fibroblast, but also has promoting actions on proliferation, migration or differentiation of various cells such as vascular endothelial cells, vascular smooth muscle cells, epithelial cells, etc. Also, it has been clarified that it has potent vascularization *in vivo*. Since bFGF has such a pharmacological action, it has been developed as a treating agent for intractable skin ulcer, excellent treatment effects and safety thereof are confirmed in clinical studies, and now, it is commercially available. bFGF also shows effective action on bone tissue and promotes healing of fracture. Moreover, it has been expected for clinical applications to various bone diseases which are required to cause osteogenesis including fracture as a new type osteogenesis promoter which induces formation of a bone tissue at a local portion to which it is administered. When bFGF is administered to a periodontium defect portion, it has been further clarified that it promotes alveolar bone formation (bone density, alveolar bone area, bone trabecular area), and promotes cementification and regeneration of a periodontal ligament at an exposed tooth root surface, whereby a periodontium can be regenerated with good balance. From the fact, it has been admitted that it is effective to obtain regeneration curing, or new attachment curing, which is a final goal for the treatment of periodontosis. Also, it has been considered to be useful for the treatment of various kinds of periodontal diseases such as repair of periodontium after extraction of a tooth, and after extraction of a cyst or a oral cavity cancer, progress of fixation of an implant material, regeneration of dentin defected by dental caries and the like. Of these, it has been studied to apply to periodontosis that is a chronic tissue defect (WO95/05840)

On the other hand, it is desired to directly administer the bFGF only to the affected part at which the action of the bFGF is required when the fact that the bFGF has a potent and various pharmacological actions is taking into consideration. To apply the bFGF to various diseases with good efficiency, a preparation plan suitable for the respective diseases is required. In fact, a preparation is optimized such that a spray preparation is for intractable skin ulcer, a gel preparation for bone diseases and the like. However, a useful bFGF preparation that can be directly applied to various kinds of periodontal diseases such as periodontosis, etc. with good efficiency has not yet been developed as of today.

On the other hand, as a preparation for dental use, a paste, a liquid agent, an ointment, a gel agent, etc. have generally been known. However, bFGF is physiochemically unstable and an effective dose is a low amount, so that a form of the agent or preparation conditions are restricted, in particular, it has been considered that development of a paste or an ointment would be difficult. On the other hand, it has been considered that a liquid agent and a gel agent each containing bFGF can be developed by making it a preparation which is of a type that is prepared at the time of use. In clinical applications of the bFGF to various kinds of periodontal diseases, particularly for the treatment of periodontosis, at the time of flap operation (an operation in which gum is opened and tartar, etc. are removed), it can be expected to administer it into the gum while expecting regeneration of alveolar bone. However, in a liquid agent, the preparation cannot be retained with a sufficient period of time to the affected part due to drip of the liquid when it is administered to the upper jaw portion, so that it is afraid that the preparation cannot be administered to the affected part with good efficiency. Thus, as a preparation that supplements the above defects, a jerry state gel preparation can be considered. The gel preparation cannot be said to be a preferred preparation form for uniform application a medical agent with a low content onto the affected part which comprises various shape quantitatively. In particular, in the case of the gel preparation, it can be expected when a base material remained at the affected part for a long period of time, it inhibits repair of a tissue, and further, it can be expected that a patient will complain of unpleasant feeling as an alien substance. Thus, the base material is required to be a high functional material in which it is rapidly decomposed or disappeared within a certain period of time after it is retained to the affected part after administration. However, such a base material has a defect that it is expensive.
EP 0 702 959 and Tabeta et al., Tissue Engineering 5(2), 1999, 127-138. describe cross-linked gelatin gel preparations containing bFGF. The use of bFGF as active ingredient for treating periodontal diseases is described in JP 7017876 and EP 0 677 294.

Accordingly, to develop the bFGF as a treatment agent for various kinds of periodontal diseases such as periodontosis, etc., it is desired that the bFGF is maintained stably, and the bFGF with a low content can be coated onto the affected part which has a small area while prohibiting liquid dripping with various shapes uniformly. Also, it has been desired to develop a preparation for dental use that is rapidly decomposed or disappeared after it is retained with an extent that it does not prohibit repair of a tissue after application, can be coated with good efficiency and comprises inexpensive materials.

The inventors of the present application have earnestly studied to solve the above-mentioned problems, and as a result, they have found that, bFGF is formulated with a thickener which can maintain a certain viscosity when it is made a solution to make a viscous preparation, whereby bFGF can be maintained stably, bFGF with a low content can be coated uniformly with a quantitative amount on a portion to be treated having various shapes, whereby a preparation excellent in local retention can be obtained, to accomplish the present invention.

### Disclosure of the invention

The present invention relates to a viscous preparation for dental use which contains a basic fibroblast growth factor (bFGF) as an effective ingredient and further a thickener as defined in claim 1.

In the viscous preparation for dental use of the present invention, by formulating a thickener as defined in claim 1 having a certain viscosity, suitable viscosity and local retention of the viscous preparation are ensured, whereby administration of a bFGF to a portion to be treated can be more ensured.

The present invention also relates to a kit for preparing the viscous preparation for dental use of the present invention, which contains a bFGF, a thickener as defined in claim 1, and, if necessary, an inactive and non-toxic additive, and a dissolving liquid, and a method for preparing the viscous preparation for dental use of the present invention, which comprises dissolving a bFGF, a thickener as defined in claim 1, and, if necessary, an inactive and non-toxic additive, in a dissolving liquid.

### Brief description of the drawings

Fig. 1 is a drawing showing a releasing curve of bFGF from the viscous preparation for dental use of the present invention and a bFGF aqueous solution,
Fig. 2 is a drawing showing a remaining ratio of ¹²⁵I-labelled bFGF in a tissue after intramuscular administration of the viscous preparation for dental use of the present invention and a bFGF aqueous solution to rats, and
Fig. 3 is a drawing showing a remaining ratio (%) of ¹²⁵I-labelled bFGF after 6 hours from the administration relative to the time immediately after the administration when the viscous preparation for dental use of the present invention or a bFGF aqueous solution is administered to rabbit mandibula defected portion.

### Best mode for carrying out the invention

The viscous preparation for dental use of the present invention is a viscous preparation for dental use containing a bFGF as an effective ingredient and further containing a thickener as defined in claim 1. The viscous preparation herein mentioned means a preparation which shows a viscosity of about 20 to 25,000 mPa·s measured at 25°C by using an E type viscometer. The viscous preparation for dental use of the present invention preferably has a viscosity of about 1,000 to 20,000 mPa·s, particularly preferably about 3,000 to 15,000 mPa·s.

As the bFGF to be contained in the viscous preparation for dental use of the present invention, those derived from mammals may be mentioned. As the mammals, there may be mentioned human, monkey, pig, bovine, sheep, horse, and the like. bFGF can be obtained by the conventionally known method from these mammals, and bFGF derived from animals, for example, bovine bFGF is commercially available as a reagent from a plural number of companies.

Also, as said bFGF, those produced by the recombinant DNA technology may be used. For producing bFGF by the recombinant DNA technology, for example, the technique disclosed in, for example, Japanese PCT Patent publication No. Sho.63-500843 can be used. Also, a human bFGF produced by the recombinant DNA technology is commercially available as a reagent, and a general name: Trafermin (genetical recombination) can be preferably used.

A bFGF concentration of the viscous preparation for dental use of the present application is, in view of the effects on the periodontal diseases, preferably about 0.0001 to 20% by weight, more preferably about 0.001 to 10% by weight, most preferably about 0.01 to 1% by weight based on the total weight of the preparation.

The thickener to be contained in the viscous preparation for dental use of the present invention is a thickener that can show the above-mentioned viscosity (about 20 to 25,000mPa·s) when it is made a solution, exert no bad effects on stability of the bFGF, and is pharmaceutically acceptable. More specifically, it is selected from hydroxypropyl cellulose, sodium alginate, propylene glycol alginate, carboxyvinyl polymer, carmelose sodium, hyaluronic acid, sodium hyaluronate, hydroxyethyl cellulose, hydroxyethylmethyl cellulose, hydroxypropylmethyl cellulose, polyacrylic acid, sodium polyacrylate, polyacrylic acid partially neutralized product, polyvinyl alcohol, methyl cellulose, xanthan gum, chondroitin acid, and sodium chondroitin sulfate, etc. may be used. Of these, when an effect of bFGF on stability is considered, hydroxypropyl cellulose (HPC), sodium hyaluronate, xanthan gum, and sodium chondroitin sulfate are preferably used, and hydroxypropyl cellulose is particularly preferably used.

In addition to these thickeners, a thickener such as gum Arabic, gum Arabic powder, guaiac gum, glucono-δ-lactone, gelatin, dextran 70, dextrin, tragacanth, tragacanth powder, povidone, starch syrup, rosin, polyoxyethylene (160) polyoxypropylene (30) glycol, polyoxyethylene (200) polyoxypropylene glycol (70), and a copolymer of methyl vinyl ether and maleic anhydride may be used.

In the viscous preparation for dental use of the present invention, a hydroxypropyl cellulose particularly preferably used as a thickener is a hydroxypropyl ether derivative of cellulose, preferred are those containing 53.4 to 77.5% of a hydroxypropyl group (The Japanese Pharmacopoeia Fourteenth Edition D) when a dried material is determined. When HPC is dissolved in water, it becomes a viscous liquid, any HPC showing a viscosity of about 20 to 25,000 mPa·s when it is made an aqueous solution and measured the viscosity at 25°C by using an E type viscometer and having an optional molecular weight can be used with a concentration showing the above-mentioned viscosity. However, that having a molecular weight of about 100,000 to 500,000 which shows high thickening property with a low concentration can be preferably used, and more preferably that having 110,000 to 400,000. For example, when HPC with a molecular weight of about 110,000 to 150,000 is used, HPC-M available from NIPPON SODA CO., LTD. can be used preferably with a ratio of about 2 to 18% by weight, more preferably with a ratio of about 3 to 10% by weight based on the whole preparation. Also, when HPC with a molecular weight of about 250,000 to 400,000 is used, HPC-H available from NIPPON SODA CO., LTD. can be used preferably with a ratio of about 1 to 9% by weight, more preferably with a ratio of about 2 to 6% by weight. It is also possible to use HPCs having different molecular weights in combination by optionally mixing within the range in which the mixture accomplishes the above-mentioned viscosity.

Also, when sodium hyaluronate, xanthan gum, or sodium chondroitin sulfate is used as a thickener, they can be used with a concentration in the range that can accomplish the above-mentioned viscosity. For example, when sodium hyaluronate is used with a molecular weight of about 600,000 to 900,000, it can be used with a concentration of about 1% by weight. When xanthan gum is used with a molecular weight of about 2,000,000, it can be used with a concentration of about 1% by weight. With regard to the other thickeners, they can be used with a concentration in the range that they can accomplish the above-mentioned viscosity.

In the viscous preparation for dental use of the present invention, various kinds of pharmaceutically acceptable additives such as a sugar, a pH controller, a preservative, a chelating agent, an emulsifier, a suspending agent, a stabilizer, a colorant, etc. may be further contained, if necessary. As the sugar, there may be mentioned sucrose, trehalose, etc., and sucrose can be particularly preferably used. Also, a pH of the viscous preparation for dental use of the present invention is desirably maintained to about 4.5 to 8, particularly to 4.5 to 6.5, and as a pH controller to maintain the pH, there may be mentioned a buffer comprising citric acid and sodium citrate or acetic acid and sodium acetate. As a preservative and a chelating agent, there may be mentioned benzalkonium chloride and sodium edentate, respectively.

The viscous preparation for dental use of the present invention can be prepared by formulating the above-mentioned thickener to bFGF, and dissolving in a dissolving solution to make a solution having a predetermined viscosity. A ratio of the bFGF based on the whole preparation is, as mentioned above, preferably about 0.0001 to 20% by weight, more preferably about 0.001 to 10% by weight, most preferably about 0.01 to 1% by weight, and the bFGF is so formulated that it becomes such a ratio. Also, as the dissolving solution, water can be preferably used. A ratio of the thickener to be used based on the whole preparation may vary depending on the kind of the thickener to be used, and can be determined within the range that shows a viscosity of about 20 to 25,000 mPa·s in the state of a solution. For example, when a HPC having a molecular weight of about 110,000 to 150,000 is used as a thickener, the thickener is so dissolved in the dissolving solution that it becomes a ratio of about 2 to 18% by weight, preferably a ratio of about 3 to 10% by weight. Also, when a HPC having a molecular weight of about 250,000 to 400,000 is used, the thickener is so dissolved in the dissolving solution that it becomes a ratio of about 1 to 9% by weight, preferably a ratio of about 2 to 6% by weight.

For preparing the viscous preparation for dental use of the present invention, for example, by using the method in which an aqueous solution of bFGF is added to a powder state HPC, and mixing them, or HPC is dissolved in water to obtain an HPC viscous aqueous solution, and an aqueous solution of bFGF is mixed thereto, whereby it can be optionally prepared. With regard to the specific method for preparation, it is specifically described in various manners in the following Examples, but the present invention is not limited to these, and optionally prepared within the range of technical common sense in this field of the art. Moreover, the viscous preparation for dental use of the present invention prepared as mentioned above can be prepared to ensure a long term stability of the bFGF by lyophilizing the preparation, and preparing again with addition of water at the time of use. Also, the bFGF may be preserved in a lyophilized state, and at the time of use, it may be prepared by adding a viscous aqueous solution of a HPC.

Accordingly, the present invention also relates to a kit for preparing the viscous preparation for dental use of the present invention which comprises bFGF and a thickener, and if necessary, an inactive and non-toxic additive, and a dissolving solution, and to a process for preparing the viscous preparation for dental use of the present invention, which comprises dissolving bFGF, a thickener, and if necessary, an inactive and non-toxic additive in a dissolving solution.

Also, the viscous preparation for dental use of the present invention is required to be in a sterilized state depending on the method of application, so that, at the time of preparation, it can be maintained in a sterilized state by previously filtering and sterilizing a bFGF solution, subjecting a HPC powder to irradiation sterilization or dry sterilization, and by subjecting a HPC viscous liquid to autoclave sterilization, whereby a sterilized state can be ensured.

The viscous preparation for dental use of the present invention prepared by the above-mentioned method can be directly administered to an affected part of various kinds of periodontal diseases in the same manner as in an ointment, a gel agent, a paste and a liquid agent, etc. For example, a suitable amount of the viscous preparation is taken by a 2 ml of an injection syringe attached with a needle having a diameter of 18G or so, and administered with the needle of 18G when it is administered to the affected part broadly with a large amount. Also, when it is administered to a small gap such as a periodontal pocket, the needle is replaced with a needle of 26G or so, and then the preparation can be administered. It is also possible that the preparation is previously filled in a reservoir portion of a kit product such as a simplified injection syringe, and then, it is administered.

Also, it is possible to apply the preparation directly or with a spatula onto the affected part. In addition to the above, by measuring a necessary amount using a pressure type quantitative pump, and the preparation can be applied directly or with a spatula onto the affected part.

A dose of the viscous preparation for dental use of the present invention to be administered may be optionally changed depending on a kind of the periodontal diseases to be applied, seriousness, a range of the affected part, sex or a body weight of a patient, and the like, and generally, in the case of a human, the preparation is preferably applied to the affected part in a dose so that the bFGF is applied in a dose of about 0.1 to 3000 µg, preferably 1 to 1500 µg per administration.

The viscous preparation for dental use of the present invention can be applied for the purpose of treatment or prophylaxis of periodontal diseases not only periodontosis as mentioned above but also repair of periodontium after extract of a tooth and after removal of a cyst or oral cavity cancer, progress of fixation of implanting material, regeneration of dentin defected by dental caries, and the like. For example, in the case of the periodontosis, it can be directly administered to the affected part, and when a tooth root surface is exposed by a flap operation, the viscous preparation for dental use of the present invention is applied or injected on the exposed surface. A number of administration may vary depending on a kind of the periodontal diseases, a degree of seriousness, etc., and for example, when it is used in a flap operation in periodontosis, the present preparation is administered only once since it is administered to the affected part such as the tooth root surface, etc., and the affected part is sewed.

### Example

In the following, the viscous preparation for dental use of the present invention will be explained in more detail, but the present invention is not limited by these. Incidentally, as the human bFGF to be used in the following Examples and Test Examples, Trafermin (genetical recombination) was used.

### Example 1

To 485 ml of water was gradually added HPC (HPC-H (available from Nippon Soda Co., Ltd.), 15.0 g), and stirring was continued until the particles were completely dispersed and dissolved to obtain an HPC viscous liquid. After apportioned in an ampoule with each 2 ml, it was sealed by melting and sterilized in an autoclave. The resulting HPC viscous liquid had a viscosity of 10082.0 mPa·s (measured by using an E type viscometer at 25°C. Also, in the following Examples and Test Examples, the viscosity was measured in the same conditions as in Example 1 otherwise specifically mentioned). Separately, a citrate/sucrose buffer (pH 5.1, 1.0 ml) each containing 0.89 or 2.67 mg/ml of human bFGF was filtered and sterilized, and lyophilized in a vial. To these lyophilized product was added the above-mentioned sterilized HPC viscous liquid (1.0 ml), the mixture was stirred, allowed to stand for degassing to obtain a viscous preparation for dental use of the present invention (each was made Preparation example 1a (0.89 mg/ml) and Preparation example 1b (2.67 mg/ml)).

### Example 2

To 87 ml of water were gradually added HPC (HPC-H (available from Nippon Soda Co., Ltd.), 3.0 g) and 9.0 g of sucrose, and stirring was continued until the particles were completely dispersed and dissolved to obtain an HPC viscous liquid. This HPC viscous liquid was cooled while stirring, and a citrate buffer (pH 5.1, 1.0 ml) containing 10.1 mg/ml of human bFGF was gradually added thereto, and stirred until the mixture became uniform to obtain a viscous preparation for dental use of the present invention.

### Example 3

A citrate/sucrose buffer (pH 5.1, 1.0 ml) each containing 0.89, 2.67 or 8.00 mg/ml of human bFGF was filtered and sterilized, and lyophilized in a vial. To this lyophilized product was added 1 ml of water for injection to dissolve the product, and then, HPC (HPC-H (available from Nippon Soda Co., Ltd.), 30 mg) was gradually added to dissolve in the mixture to obtain a viscous preparation for dental use of the present invention.

### Example 4

A citrate/sucrose buffer (pH 5.1, 1.0 ml) each containing 0.89, 2.67 or 8.00 mg/ml of human bFGF was filtered and sterilized, and HPC (HPC-H (available from Nippon Soda Co., Ltd.), 30 mg) was gradually added thereto and after dissolution, the mixture was lyophilized in a vial. Water (1 ml) was added to this lyophilized product to obtain a viscous preparation for dental use of the present invention.

### Example 5

A citrate/sucrose buffer (pH 5.1, 1.0 ml) each containing 0.89, 2.67 or 8.00 mg/ml of human bFGF was filtered and sterilized, and the mixture was lyophilized in a vial. Separately, after HPC (HPC-H (available from Nippon Soda Co., Ltd.), 30 mg) was gradually dissolved with water (1 ml), the mixture was lyophilized in a vial. Water (each 0.5 ml) was added to these lyophilized products, and both materials were mixed to obtain a viscous preparation for dental use of the present invention.

### Example 6

A citrate/sucrose buffer (pH 5.1, 1.0 ml) each containing 0.89, 2.67 or 8.00 mg/ml of human bFGF was filtered and sterilized, and HPC (HPC-H (available from Nippon Soda Co., Ltd.), 30 mg) was gradually added thereto to dissolve the material to obtain a viscous preparation for dental use of the present invention.

### Example 7

Water (1 ml) was added to HPC (HPC-H (available from Nippon Soda Co., Ltd.), 60 mg) to dissolve therein, and the solution was added to a filtered and sterilized citrate/sucrose buffer (pH 5.1, 1.0 ml) containing 5.34 mg/ml of human bFGF and mixed to obtain a viscous preparation for dental use of the present invention.

### Example 8

After HPC (HPC-H (available from Nippon Soda Co., Ltd.), 30 mg) was gradually dissolved in water (1 ml), the solution was lyophilized in a vial. To the lyophilized product was added a filtered and sterilized citrate/sucrose buffer (pH 5.1, 1.0 ml) each containing 0.89, 2.67 or 8.00 mg/ml of human bFGF to dissolve the mixture and to obtain a viscous preparation for dental use of the present invention.

### Example 9

After HPC (HPC-H (available from Nippon Soda Co., Ltd.), 60 mg) was gradually dissolved in water (1 ml), the solution was lyophilized in a vial. To the lyophilized product was added a filtered and sterilized citrate/sucrose buffer (pH 5.1, 1.0 ml) each containing 0.89, 2.67 or 8.00 mg/ml of human bFGF to dissolve the mixture and to obtain a viscous preparation for dental use of the present invention.

### Test Example 1

Stability of the bFGF in the viscous preparation for dental use of the present invention was investigated.

Preparations (1a and 1b) obtained in Example 1 were preserved in a thermostat chamber at 25°C, and a remaining ratio of the bFGF was measured by the HPLC method with a lapse of time. The results are shown in Table 1.

**Table 1**

| Time (hr) | bFGF remaining ratio (%) | |
|---|---|---|
| | Preparation 1a | Preparation 1b |
| 8 | 98.7 | 98.6 |
| 24 | 98.1 | 99.6 |
| 42 | 99.1 | 98.9 |

From the results shown in Table 1, the viscous preparations for dental use of the present invention were confirmed to retain bFGF stably at 42 hours after the preparation.

### Test Example 2

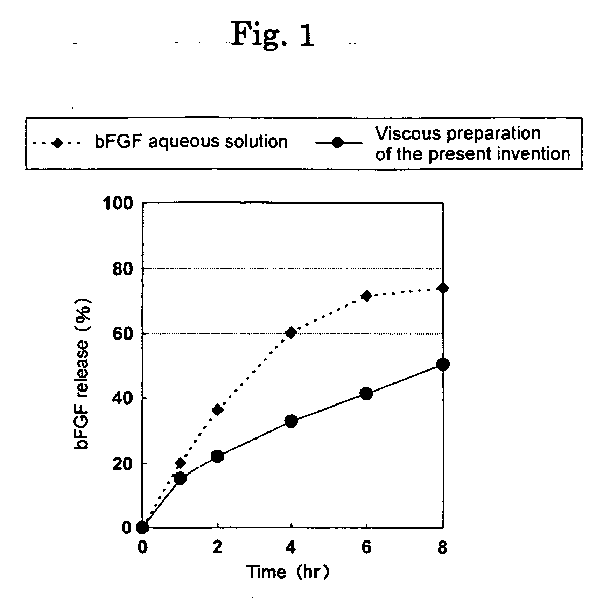

By using a Frantz type diffusion cell, a drug releasing property of Preparation (1b) obtained in Example 1 was investigated as a bFGF releasing ratio of the diffusion cell to a receptor phase, in comparison with an aqueous solution of the bFGF (bFGF concentration: 0.267% by weight) as a control. An amount of the bFGF in the receptor phase which passes through a membrane made of cellulose was determined by the HPLC method with a lapse of time to obtain the bFGF releasing ratio. As the receptor solution, a citrate/sucrose buffer was used. The results are shown in Fig. 1.

According to the results shown in Fig. 1, the viscous preparation for dental use of the present invention showed a tendency that it can store the bFGF at a local portion for a long period of time than in the bFGF aqueous solution, and showed a pattern in which bFGF was released with a constant rate for a long period of time.

### Test Example 3

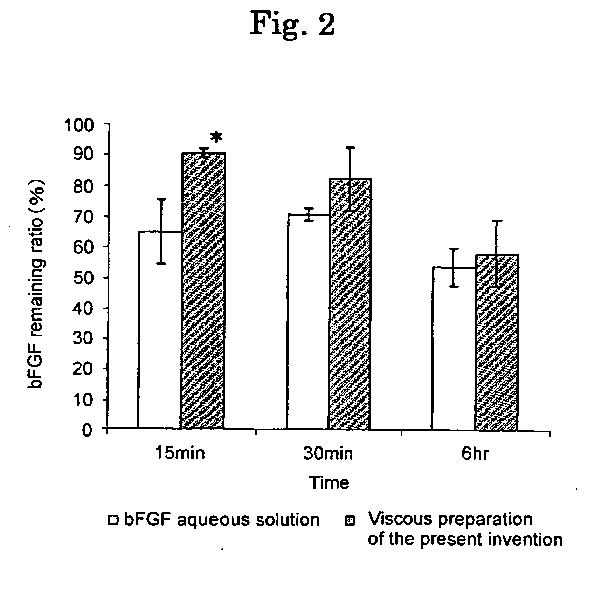

The viscous preparation for dental use of the present invention (bFGF concentration: 0.97 mg/ml; HPC concentration: 3%; viscosity: 10423 mPa·s) containing ¹²⁵I-labelled bFGF or a ¹²⁵I-labelled bFGF aqueous solution was intramuscularly administered (50 µl) to the neighbor of fibula at the left hind leg of SD series male rats (7 weeks old). An administered dose of the bFGF was 48.52 pg per each rat, and as a radio-labelled bFGF, it was 264.29 kBq per each rat. Fifteen minutes, 30 minutes and 6 hours after the administration, ¹²⁵I-labelled bFGF at the administered portion was measured. A remaining ratio of radioactivity in the tissue to which administered was shown in Fig. 2. The respective values show an average value of three samples ± standard deviation.

According to the results shown in Fig. 2, when the viscous preparation for dental use of the present invention is administered intramuscularly, transfer of the bFGF into a blood is slow as compared with the case where the bFGF aqueous solution is administered. In the rats to which the viscous preparation of the present invention was administered, 90.6% of the radio-labelled bFGF was remained at the administered portion at 15 minutes after the administration, and when it is compared with the case where the bFGF aqueous solution was administered, the bFGF remaining ratio at the administered portion was significantly high (0.01<p≦0.05, Student t detection) in the case where the preparation of the present invention was administered. Also, the remaining ratio of the bFGF at the administered portion was high in the preparation of the present invention than that of the aqueous solution after 6 hours from the administration.

### Test Example 4

By using a ¹²⁵I-labelled bFGF having a specific activity of about 25kBq/pg, to a defect portion of the right side mandibula of rabbits was administered 50 µl of the viscous preparation for dental use of the present invention having the composition shown in the following Table 2 or the ¹²⁵I-labelled bFGF aqueous solution, and a remaining ratio of the ¹²⁵I-labelled bFGF at 6 hours after the administration was measured.

**Table 2**

| | ¹²⁵I-labelled bFGF concentration | HPC concentration | Viscosity (mPa·s) | bFGF administered dose (µg) |
|---|---|---|---|---|
| ¹²⁵I-labelled bFGF aqueous solution | 0.99 mg/ml | 0 | 0 | 49.5 |
| Viscous preparation 1 of the present invention | 1.67 mg/ml | 1% | 53 | 83.5 |
| Viscous preparation 2 of the present invention | 1.71 mg/ml | 2% | 1126 | 85.5 |
| Viscous preparation 3 of the present invention | 1.05 mg/ml | 3% | 7898 | 52·5 |

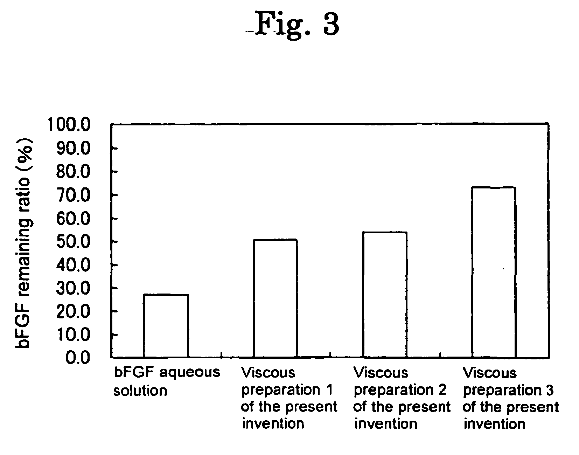

More specifically, under Nembutal anesthesia (50 mg/ml solution was administered in an amount of about 3 ml), rabbits were retained with the abdomen down, and locally anesthetized with Xylocaine (about 20 mg/ml solution was administered in an amount of about 1 ml), gum was cut from a cutting tooth to a posterior tooth along with the mandibula at the right side, and a periosteum was peeled off to prepare a two-wall type defect at a cutting tooth portion of a body of mandible at the right side mandibula (buccolingual width: about 4 mm; mesiodistal width: about 8 mm; Depth: about 4 mm). After hemostasis, 50 µl of the viscous preparation for dental use of the present invention or the ¹²⁵I-labelled bFGF aqueous solution was administered to the defect portion at the right side mandibula, and after allowing to stand for 30 seconds, the cut gum was sutured by putting it on the defect portion. After 6 hours from the administration, whole blood was collected, the right side mandibula (from the right cutting tooth including the defect portion to before the premolar tooth) and the cut and sutured gum were extracted, and radioactivity was measured by a γ counter. A remaining ratio (%) of the ¹²⁵I-labelled bFGF after 6 hours from the administration based on immediately after the administration is shown in Table 3 as well as in Fig. 3.

**Table 3**

| Remaining ratio (%) of ¹²⁵I-labelled bFGF at rabbit mandibula defect portion | | | |
|---|---|---|---|
| ¹²⁵I-labelled bFGF aqueous solution | Viscous preparation 1 of the present invention | Viscous preparation 2 of the present invention | Viscous preparation 3 of the present invention |
| 27.2 | 50.7 | 54.0 | 73.0 |

From the above-mentioned results, as compared with the case where bFGF aqueous solution was administered, when the viscous preparation of the present invention was administered, it could be admitted that bFGF was remained at the administered portion, particularly at the right gum with a high concentration even after 6 hours from the administration, and the remaining ratio was high as the HPC concentration contained in the viscous preparation of the present invention was high. From these results, in the viscous preparation of the present invention, it could be shown that the bFGF could be remained at the administered portion with a high concentration over a long period of time after the administration as compared with the bFGF aqueous solution.

### Utilizable field in industry

The viscous preparation for dental use of the present invention contains a basic fibroblast growth factor (bFGF) as an effective ingredient, and further contains a thickener as defined in claim 1, whereby it has a certain degree of a viscosity. As a result, the bFGF as an effective ingredient can be physiochemically retained stably, and when it is applied to an affected part as a treatment agent of various kinds of periodontal diseases such as periodontosis, etc., it can be uniformly applied onto the diseased portion, and stimulation or an alien feeling to the affected part is a little. The applied preparation stays for a relatively longer period of time without flowing down from the applied portion, whereby the bFGF contained in the preparation is released to the affected part stably, and as a result, excellent healing effects can be obtained. In addition, the preparation of the present invention has fluidity, so that it corresponds to unevenness or gap of the diseased portion and can be administered precisely.

## Claims

1. A viscous preparation for dental use which comprises basic fibroblast growth factor (bFGF) as an effective ingredient and further a thickener, which is at least one selected from the group consisting of hydroxypropyl cellulose, sodium alginate, propylene glycol alginate, carboxyvinyl polymer, carmelose sodium, hyaluronic acid, sodium hyaluronate, hydroxyethyl cellulose, hydroxyethylmethyl cellulose, hydroxypropylmethyl cellulose, polyacrylic acid, sodium polyacrylate, polyacrylic acid partial neutralized product, polyvinyl alcohol, methyl cellulose, xanthan gum, chondroitin sulfuric acid, and sodium chondroitin sulfate.

2. The viscous preparation for dental use according to claim 1, wherein the thickener is hydroxypropyl cellulose.

3. The viscous preparation for dental use according to claim 1 or 2, wherein the amount of bFGF is 0.0001 to 20% by weight based on the total weight of the preparation.

4. A viscous preparation for dental use according to any one of claims 1 to 3 for use in the treatment of periodontosis.

5. A kit for preparing the viscous preparation for dental use according to any one of Claims 1 to 4, which comprises bFGF, a thickener as defined in claim 1, and, if necessary, an inactive and non-toxic additive and a solution for dissolution.

6. A method for preparing the viscous preparation for dental use according to any one of Claims 1 to 4, which comprises dissolving bFGF, a thickener as defined in claim 1, and, if necessary, an inactive and non-toxic additive into a solution for dissolution.

7. The method according to Claim 6, wherein the solution for dissolution is water.

## Patentansprüche

1. Viskose Zubereitung für die dentale Anwendung, welche den basischen Fibroblastenwachstumsfaktor (bFGF) als Wirkstoff und darüber hinaus einen Verdicker umfasst, welcher mindestens einer ist, der aus der Gruppe ausgewählt ist, die aus Hydroxypropylcellulose, Natriumalginat, Propylenglykolalginat, Carboxvinylpolymer, Natriumcarmelose, Hyaluronsäure, Natriumhyaluronat, Hydroxyethylcellulose, Hydroxyethylmethylcellulose, Hydroxypropylmethylcellulose, Polyacrylsäure, Natriumpolyacrylat, teilweise neutralisiertes Polyacrylsäureprodukt, Polyvinylalkohol, Methylcellulose, Xanthangummi, Chondroitinschwefelsäure und Natriumchondroitinsulfat besteht.

2. Viskose Zubereitung für die dentale Anwendung gemäß Anspruch 1, worin der Verdicker Hydroxypropylcellulose ist.

3. Viskose Zubereitung für die dentale Anwendung gemäß Anspruch 1 oder 2, worin die Menge an bFGF 0,0001 bis 20 Gew.% bezogen auf das Gesamtgewicht der Zubereitung ist.

4. Viskose Zubereitung für die dentale Anwendung gemäß irgendeinem der Ansprüche 1 bis 3 für die Verwendung in der Behandlung von Parodontose.

5. Kit für die Herstellung der viskosen Zubereitung für die dentale Anwendung gemäß irgendeinem der Ansprüche 1 bis 4, welches bFGF, einen Verdicker wie in Anspruch 1 definiert und, falls notwendig, einen inaktiven und nichttoxischen Zusatz und eine Lösung für die Auflösung umfasst.

6. Verfahren für die Herstellung der viskosen Zubereitung für die dentale Anwendung gemäß irgendeinem der Ansprüche 1 bis 4, welches das Auflösen von bFGF, eines Verdickers wie in Anspruch 1 definiert und, falls notwendig, eines inaktiven und nichttoxischen Zusatzes in einer Lösung für die Auflösung umfasst.

7. Verfahren gemäß Anspruch 6, worin die Lösung für die Auflösung Wasser ist.

## Revendications

1. Préparation visqueuse à usage dentaire, qui comprend du facteur de croissance des fibroblastes basique (bFGF) comme ingrédient actif et en outre un épaississant, qui est au moins un épaississant choisi dans le groupe constitué par l'hydroxypropylcellulose, l'alginate de sodium, l'alginate de propylèneglycol, un polymère carboxyvinylique, le carmellose sodique, l'acide hyaluronique, l'hyaluronate de sodium, l'hydroxyéthylcellulose, l'hydroxyéthylméthylcellulose, l'hydroxypropyl-méthylcellulose, le poly(acide acrylique), le poly(acrylate de sodium), un produit de neutralisation partielle de poly(acide acrylique), le poly(alcool vinylique), la méthylcellulose, la gomme xanthane, le sulfate de chondroïtine, et le sulfate de chondroïtine sodique.

2. Préparation visqueuse à usage dentaire selon la revendication 1, où l'épaississant est l'hydroxypropylcellulose.

3. Préparation visqueuse à usage dentaire selon la revendication 1 ou 2, où la quantité de bFGF est de 0,0001 à 20 % en masse par rapport à la masse totale de la préparation.

4. Préparation visqueuse à usage dentaire selon l'une quelconque des revendications 1 à 3, à utiliser dans le traitement de la parodontite.

5. Kit de préparation de la préparation visqueuse à usage dentaire selon l'une quelconque des revendications 1 à 4, qui comprend du bFGF, un épaississant tel que défini dans la revendication 1, et, si nécessaire, un additif inactif et non toxique et une solution pour la dissolution.

6. Procédé de préparation de la préparation visqueuse à usage dentaire selon l'une quelconque des revendications 1 à 4, qui comprend la dissolution du bFGF, de l'épaississant tel que défini dans la revendication 1, et, si nécessaire, d'un additif inactif et non toxique dans une solution pour la dissolution.

7. Procédé selon la revendication 6, dans lequel la solution pour la dissolution est l'eau.
